# EUROPÄISCHE PATENTSCHRIFT

(11) **EP 0 711 275 B1**
(45) Veröffentlichungstag und Bekanntmachung des Hinweises auf die Patenterteilung: **15.04.1998**
(21) Anmeldenummer: 94924289.5
(22) Anmeldetag: 26.07.1994
(51) Int. Cl.: C07C 279/24, C07C 275/58, C07C 275/60, C07C 335/28, C07D 213/74, C07D 333/36, A61K 31/155, A61K 31/325

(54) **(THIO)CARBAMIDINSÄURE-DERIVATE UND DIESE ENTHALTENDE ARZNEIMITTEL**
(THIO)CARBIMIDINIC ACID DERIVATIVES AND MEDICAMENTS CONTAINING THE SAME
DERIVES D'ACIDE (THIO)CARBIMIDINIQUE ET MEDICAMENTS CONTENANT CES DERIVES

(30) Priorität: 31.07.1993 DE 4325741
(43) Veröffentlichungstag der Anmeldung: 15.05.1996
(73) Patentinhaber: BOEHRINGER MANNHEIM GMBH, 68298 Mannheim (DE)
(72) Erfinder: MERTENS, Alfred, D-69198 Schriesheim (DE); KÖNIG, Bernhard, D-82335 Berg (DE); LESER, Ulrike, D-81375 München (DE)
(86) Internationale Anmeldenummer: EP9402461
(87) Internationale Veröffentlichungsnummer: WO9504034

(56) Entgegenhaltungen:
- STN-Information Service file REG RN = 88727-87-1 Chem. Abstr. Nr. 100:68027
- STN-Information Service file REG RN = 77060-38-9 Chem. Abstr. Nr. 94:175410
- STN-Information Service file REG RN = 51905-13-6 Chem. Abstr. Nr. 80:108146
- DATABASE WPI Week 6800, Derwent Publications Ltd., London, GB; AN 66-31677 & JP,B,43 008 101 (SUMITOMO CHEM CO LTD)
- DATABASE WPI Week 6800, Derwent Publications Ltd., London, GB; AN 66-31377 & JP,B,43 006 612 (SUMITOMO CHEM CO LTD)

## Beschreibung

Die vorliegende Erfindung betrifft neue Arzneimittel, die (Thio)carbamidinsäure-Derivate der Formel I enthalten. Ferner bezieht sich die Erfindung auf neue (Thio)carbamidinsäure-Derivate, Verfahren zu deren Herstellung und deren Verwendung als antivirale Mittel.

Gegenstand der Erfindung sind Arzneimittel enthaltend als Wirkstoff mindestens ein (Thio)carbamidinsäure-Derivat der Formel I 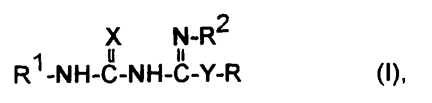 wobei
- R: einen geradkettigen oder verzweigtkettigen, gesättigten oder ungesättigten aliphatischen Rest mit 1-9 Kohlenstoffatomen, C₃-C₈-Cycloalkyl, C₁-C₆-Alkoxyalkyl, C₁-C₆-Alkylmercapto-C₁-C₆-alkyl, Amino-C₁-C₆-alkyl, C₁-C₆-alkylamino-C₁-C₆-alkyl, C₁-C₆-Dialkylamino-C₁-C₆-alkyl, Carboxy-C₁-C₆-alkyl, C₃-C₈-Cycloalkyl-C₁-C₆-alkyl; substituiertes Phenyl-C₁-C₆-alkyl; substituiertes Pyridyl-C₁-C₆-alkyl;
Phenyl, das gegebenenfalls ein- oder mehrfach durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁-C₆-Alkylcarbonylamino, C₁-C₆-Alkylaminocarbonyl, C₁-C₆-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl substituiert sein kann;
einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollständig hvdriert sein können,
- R¹: Phenyl, Phenyl-C₁-C₆-alkyl, Hetaryl oder Hetaryl-C₁-C₆-alkyl sein kann, wobei die Heteroatome gleich oder verschieden sein können und Sauerstoff, Schwefel oder Stickstoff bedeuten, die gegebenenfalls ein- oder mehrfach durch einen geradkettigen oder verzweigten, gesättigten oder ungesättigten C₁-C₆- bzw. C₂-C₆-Alkylrest, C₁-C₆-Alkoxy, C₁-C₆-Alkylmercapto, C₁-C₆-Alkylsulfinyl, C₁-C₆-Alkylsulfonyl, C₁-C₆- Alkylamino, C₁-C₆-Dialkyl- amino, C₁-C₆-Alkoxycarbonyl, Carboxy, Car- boxy-C₁-C₆-alkylcarbonyl, Carboxy-C₂-C₆-alkenylcarbonyl, Amino, Halogen, Hydroxy, Nitro, Cyano, Azido, Phenyl oder Benzyloxy substituiert sein können,
- R²: Wasserstoff, C₁-C₆-Alkyl, C₁-C₆-Alkoxy, Cyano, Amino oder Hydroxy,
- X: Sauerstoff, Schwefel oder NR² und
- Y: Sauerstoff oder Schwefel bedeuten,
sowie deren Tautomere, Enantiomere, Diastereomere und physiologisch verträgliche Salze,
mit der Maßgabe, daß R¹ nicht einen 2,6 methylsubstituierten Phenylring darstellt, wenn X = O, Y = S, R = Methyl und R² = H ist.

Substanzen der Formel I, in der R C₁-C₄-Alkyl, Allyl, Benzyl, 4-Bromophenylmethyl oder Phenyl bedeutet und R¹ einen unsubstituierten oder substituierten Phenylring darstellt, sind bereits aus der Literatur bekannt. Dabei wurden diese Substanzen vorwiegend als Zwischenprodukte zur Herstellung von heterocyclischen Endprodukten eingesetzt.

In Synthesis 1020, 1990 wird die oxidative Cyclisierung einiger Substanzen der Formel I beschrieben. Die Herstellung von Triazinen aus den genannten Verbindungen wird erwähnt in DE 3,516,631; US 4,460,585; US 3,914,224; und Chem. Ber. 105, 3168, 1972. Die Herstellung von Amidinoharnstoffen aus den zuvor genannten Substanzen ist beschrieben in IN 152552, (1984), sowie die Herstellung von Thiatriazinen in J. Het. Chem. 21, 1553, 1984. In J. Plant. Growth Regul. 2, 129, 1983 wird einigen Verbindungen der Formel I eine Stimulation des Reis- wachstums zugeschrieben. Mehrere Literaturstellen erwähnen diese Verbindungen zur Herstellung von Thiadiazolen, Dithiazolidinen und Thiadiazolidinen (Indian J. Chem., Sect. B, 15B, 192, 1972; Ind. J. Chem. 12, 134, 1974; J. Chem. Soc. 4191, 1962; J. Indian Chem. Soc. 38, 988, 1961; J. Chem. Soc. 1064, 1959; J. Chem. Soc. 379, 1958 und Chem. & Ind. 1482, 1956). Weiterhin ist die Herstellung einiger Verbindungen der Formel I beschrieben in Zh. Vses. Khim. Obshchest. 19, 109. 1974, Belg. 667875, 1966 und J. Indian Chem. Soc. 38, 979, 1961. Zur Herstellung von Triazolol[1.5-alpha]pyrimidinen bzw. von Triazolen werden die Substanzen in J. Het. Chem. 10, 631, 1973 bzw. J. Chem. Soc. C. 2471, 1967 zitiert. Die allgemeine Wirkung als Herbizid wird schließlich in der Anmeldung US 2,780,535 beansprucht. Eine pharmakologische Wirkung dieser Verbindungen war jedoch bisher nicht bekannt. Aus JP-B-43008101 und JP-B-43006612 sind antiviral wirkende Amidinharnstoff-Derivate bekannt.

Die Verbindungen der Formel I weisen wertvolle pharmakologische Eigenschaften auf. Insbesondere besitzen sie eine ausgeprägte antivirale Wirkung und eignen sich insbesondere zur Behandlung von durch virale oder retrovirale Infektionen verursachten Erkrankungen. Besonders eignen sie sich zur Therapie und Prophylaxe von Infektionen, die durch DNA-Viren wie z. B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-lmmunschwäche-Virus HIV-1 und -2, verursacht werden. Vor allem erscheinen die Verbindungen der Formel I geeignet zur Behandlung der klinischen Manifestationen der retroviralen HIV-Infektion beim Menschen, dem fortgeschrittenen Stadium des AIDS-verwandten Komplex (ARC) und dem klinischen Vollbild von AIDS.

Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrome (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir^{R}, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J. Infec. Dis. 157, 427-431). Die Verbindungen der allgemeinen Formel I besitzen diese Nachteile nicht. Sie wirken antiviral, ohne in pharmakologisch relevanten Dosen cytotoxisch zu sein.

Es konnte nun nachgewiesen werden, daß Verbindungen der allgemeinen Formel I die Vermehrung von DNA- bzw. RNA-Viren auf der Stufe der virusspezifischen DNA-bzw. RNA-Transkription hemmen. Die Substanzen können über die Inhibierung des Enzyme-Reverse Transcriptase die Vermehrung von Retroviren beeinflussen (vgl. Proc. Natl. Acad. Sci. USA 83, 1911, 1986 bzw. Nature 325, 773, 1987).

Gegenstand der Erfindung sind auch neue (Thio)carbamidinsäure-Derivate der Formel I 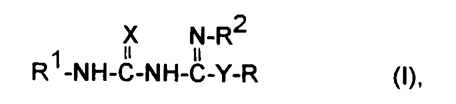 wobei
- R: einen geradkettigen oder verzweigtkettigen, gesättigten oder ungesättigten aliphatischen Rest mit 1 - 9 Kohlenstoffatomen, C₃-C₈-Cycloalkyl, C₁-C₆-Alkoxyalkyl, C₁-C₆-Alkylmercapto-C₁-C₆-alkyl, Amino-C₁-C₆-alkyl, C₁-C₆-alkylamino-C₁-C₆-alkyl, C₁-C₆-Dialkylamino-C₁-C₆-alkyl, Carboxy-C₁-C₆-alkyl, C₃-C₈-Cycloalkyl-C₁-C₆-alkyl; substituiertes Phenyl-C₁-C₆-alkyl; substituiertes Pyridyl-C₁-C₆-alkyl;
Phenyl, das gegebenenfalls ein- oder mehrfach durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁-C₆-Alkylcarbonylamino, C₁-C₆-Alkylaminocarbonyl, C₁-C₆-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl substituiert sein kann;
einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollständig hydriert sein können,
- R¹: Phenyl, Phenyl-C₁-C₆-alkyl, Hetaryl oder Hetaryl-C₁-C₆-alkyl sein kann, wobei die Heteroatome gleich oder verschieden sein können und Sauerstoff, Schwefel oder Stickstoff bedeuten, die gegebenenfalls ein- oder mehrfach durch einen geradkettigen oder verzweigten, gesättigten oder ungesättigten aliphatischen Rest, C₁-C₆- Alkoxy, C₁-C₆-Alkylmercapto, C₁-C₆-Alkylsulfinyl, C₁-C₆-Alkylsulfonyl, C₁-C₆-Alkylamino, C₁-C₆-Dialkyl- amino, C₁-C₆-Alkoxycarbonyl, Carboxy, Carboxy-C₁-C₆-alkylcarbonyl, Carboxy-C₂-C₆-alkenylcarbonyl, Amino, Halogen, Hydroxy, Nitro, Cyano, Azido, Phenyl oder Benzyloxy substituiert sein können,
- R²: Wasserstoff, C₁-C₆-Alkyl, C₁-C₆-Alkoxy, Cyano, Amino oder Hydroxy,
- X: Sauerstoff, Schwefel oder NR² und
- Y: Sauerstoff oder Schwefel bedeuten,
sowie deren Tautomere, Enantiomere, Diastereomere und physiologisch verträgliche Salze,
mit der Maßgabe, daß R nicht C₁-C₄-Alkyl, Allyl, Benzyl, 4-Bromophenylmethyl oder Phenyl bedeutet, wenn R¹ einen unsubstituierten oder substituierten Phenylring darstellt.

Ein aliphatischer Rest R in Verbindungen der allgemeinen Formel I bedeutet einen geradkettigen oder verzweigten Alkyl-, Alkenyl- oder Alkinylrest mit 1-9, vorzugsweise 2-7 Kohlenstoffatomen, wie z.B. der Propyl-, Isopropyl-, Butyl-, Isobutyl-, Pentyl-, Hexyl- oder Heptylrest. Als ungesättigte Reste kommen C₂-C₇-Alkenyl- und Alkinylreste in Frage, bevorzugt C₂-C₅, wie z.B. der Allyl-, Dimethylallyl-, Butenyl-, Isobutenyl-, Pentenyl- oder Propinylrest. Als C₃-C₈-Cycloalkylrest wird vorzugsweise Cyclopentyl und Cyclohexyl verwendet. Bei C₁-C₆-Alkoxyalkyl, C₁-C₆-Alkylmercaptoalkyl, Aminoalkyl, C₁-C₆-Alkylaminoalkyl, C₁-C₆-Dialkylaminoalkyl und Carboxyalkyl besitzt der Alkylrest 1-6 Kohlenstoffatome, vorzugsweise 1-3Kohlenstoffatome. Im Falle von C₃-C₈-Cycloalkylalkyl, Phenyl-C₁-C₃-alkyl und Pyridoxyl-C₁-C₃-alkyl ist Cyclopenthylmethyl, Benzyl, Phenethyl, Pyridylmethyl und Pyridylethyl bevorzugt.

Bedeutet R einen Phenylring, so kann dieser ein-, zwei- oder dreifach substituiert sein. Die Substituenten können unabhängig voneinander in o-, m- oder p-Stellung stehen.

Ein carbocyclischer Ring mit 7-15 C-Atomen kann mono-, bi- oder tricyclisch sein und pro Ring jeweils 5 oder 6 C-Atome aufweisen. Dieser Ring kann gesättigt, ungesättigt, teilweise gesättigt oder aromatisch sein. Beispielhaft genannt seien die folgenden Ringsysteme: der Naphthyl-, Anthracenyl-, Phenanthrenyl-, Flourenyl-, Indenyl-, Acenaphthylenyl-, Norbornyl-, Adamantylring oder eine C₃-C₇-Cydoalkyl- oder C₅-C₈-Cycloalkenylgruppe. Der carbocyclische Ring kann darüber hinaus mono- oder disubstituiert sein, wobei die Substituenten unabhängig voneinander bevorzugt in o- oder m-Stellung stehen können und ausgewählt sind aus der Gruppe C₁-C₆-Alkyl, C₁-C₆- Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₃-C₆-Alkenyloxy, C₁-C₆- Alkylamino, C₁-C₆-Dialkylamino-, C₁-C₆-Alkylcarbonylamino, C₁-C₆-Alkylaminocarbonyl, C₁-C₆-Alkoxycarbonyl-, Amino, Hydroxy, Nitro, Azido, Trifluormethyl, Cyano oder Halogen.

Die heterocyclischen mono-, bi- oder tricyclischen Ringsysteme des Restes R enthalten pro Ring 5 oder 6 Kohlenstoffatome, wobei 1-4 bzw. 1-5 C-Atome durch die Heteroatome Sauerstoff, Schwefel und/der Stickstoff ersetzt sein können. Die Ringsysteme können aromatisch, partiell oder vollständig hydriert sein. Beispielhaft genannt seien die folgenden Ringsysteme: das Pyridin-, Pyrimidin-, Pyridazin-, Pyrazin-, Triazin-, Pyrrol-, Pyrazol-, Imidazol-, Triazol-, Thiazol-, Oxazol-, Isoxazol-, Oxadiazol-, Furazan-, Furan-, Thiophen-, Indol-, Chinolin-, Isochinolin-, Cumaron-, Thionephthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei die ungesättigten bzw. aromatischen Carbo- und Heterocyclen partiell oder vollständig hydriert sein können. Das heterocyclische Ringsystem kann darüber hinaus mono- oder disubstituiert sein, wobei die Substituenten unabhängig voneinander bevorzugt in o- oder m-Stellung stehen können.

R bedeutet bevorzugt unsubstituiertes Phenyl oder Phenyl ein- oder zweifach substituiert durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₄-Alkenyl, C₂-C₆-Alkinyl, C₃-C₄-Alkenyloxy, C₁-C₆-Alkylamino, C₁-C₆-Dialkylamino-, C₁-C₆-Alkylcarbonylamino, C₁-C₆-Alkylaminocarbonyl, C₁-C₆-Alkoxycarbonyl-, Amino, Hydroxy, Nitro, Azido, Trifluormethyl, Cyano oder Halogen. Die "Alkyl"-teile der zuvor genannten Substituenten enthalten bevorzugt bis zu drei Kohlenstoffatome.

Heterocyclische Ringsysteme bzw. die Hetarylreste sind bevorzugt Pyrrol, Imidazol, Furan, Thiophen, Pyridin, Pyrimidin, Thiazol, Triazin, Indol, Chinolin, Isochinolin, Cumaron, Thio- naphthen, Benzimidazol, Chinazolin, Methylendioxybenzol, Ethylendioxybenzol, Carbazol, Acridin und Phenothiazin, wobei die heterocyclischen Ringe ein- oder zweifach substituiert sein können durch C₁-C₆-Alkyl, C₁-C₆-Alkoxy, C₁-C₆- Alkylmercapto, C₁-C₆-Alkylsulfinyl, C₁-C₆-Alkylsulfonyl, C₂-C₄-Alkenyl, C₂-C₆-Alkinyl, C₃-C₆-Alkenyloxy, C₁-C₆-Alkylamino, C₁-C₆-Dialkylamino-, C₁-C₆-Alkylcarbonylamino, C₁-C₆-Alkylaminocarbonyl, C₁-C₆-Alkoxycarbonyl-, Amino, Hydroxy, Nitro, Azido, Trifluormethyl, Cyano oder Halogen. Die "Alkyl"-teile der zuvor genannten Substituenten enthalten bevorzugt bis zu drei Kohlenstoffatome.

Bedeutet R¹ eine Phenylring oder einen aromatischen, heterocyclischen Ring, so können diese bevorzugt ein- oder mehrfach mit C₁-C₃-Alkyl, C₂-C₄-Alkenyl, C₂-C₄-Alkinyl, C₁-C₃-Alkoxy, C₁-C₃-Alkylmerapto, C₁-C₃-Alkylamino, C₁-C₃-Dialkylamino, C₁-C₃-Alkoxycarbonyl, Carboxyethylcarbonyl, Carboxyvinylcarbonyl, Amino, Halogen, Hydroxy, Cyano und Azido substituiert sein und der aromatische, heterocyclische Ring kann 3-7 Kohlenstoffatome besitzen, wobei bis zu 4 dieser Ringatome durch die Heteroatome Sauerstoff, Schwefel und/der Stickstoff ersetzt sein können. Beispielhaft seien die folgenden Heterocyclen genannt: der Furan-, Oxazol-, Isoxazol-, Oxadiazol-, Triazol-, Pyridin-, Pyridazin-, Pyrimidin- oder Pyrazin-Ring.

Besonders bevorzugte Reste für R sind C₃-C₆-Alkyl, C₂-C₆-Alkenyl, C₂-C₄-Alkinyl, Benzyl, Phenethyl, Pyridylmethyl, Pyridylethyl, gegebenenfalls mono- oder disubstituiert mit C₁-C₃-Alkyl, C₁-C₃-Alkoxy oder Halogen, Phenyl, durch C₁-C₃-Alkyl, C₁-C₃-Alkoxy, C₁-C₃-Alkylmercapto, Allyl, Allyloxy, C₁-C₃-Alkylamino, Di-C₁-C₃-alkylamino, Amino, Hydroxy, Azido, Trifluormethyl, Cyano oder Halogen mono- oder disubstituiertes Phenyl bzw. durch Methyl oder Halogen trisubstituiertes Phenyl, Naphthyl, Anthracenyl, Indenyl, Acenaphthylenyl, Phenanthrenyl, Adamantyl, Cyclohexal, Cyclohexenyl, Furyl, Thienyl, Pyridyl, Pyrimidinyl, Thiazolyl, Indolyl, Chinolinyl, Benzimidazolyl, Methylendioxyphenyl, Carbazolyl und Phenothiazinyl und durch Methyl oder Halogen mono- oder disubstituierte Derivate der vorgenannten carbocyclischen oder heterocyclischen Ringe.

Besonders bevorzugte Reste für R¹ sind Phenyl und aromatische, stickstoffhaltige Ringe mit 5-6 Ringatomen, die ein- oder mehrfach substituiert sind mit Wasserstoff, Methyl, Ethyl, Isopropyl, Allyl, Methoxy, Ethoxy, Methylmercapto, Ethylmercapto, Methylamino, Methoxycarbonyl, Ethoxycarbonyl, Carboxyvinylcarbonyl, Amino, Azido, Cyano, Hydroxy und Halogen, wobei Chlor und Brom für Halogen besonders bevorzugt sind.

Für R² ist Wasserstoff, Amino und Hydroxy besonders bevorzugt.

Für X kommt besonders bevorzugt Sauerstoff und Schwefel in Frage.

Y bedeutet bevorzugt Schwefel.

Die erfindungsgemäßen Verbindungen der allgemeinen Formel I werden nach literaturbekannten Verfahren hergestellt, indem man Isocyanate oder Thioisocyanate der allgemeinen Formel II

R¹ - N = C = X (II)

in der R¹ die oben angegebene Bedeutung hat und X Sauerstoff oder Schwefel ist, mit Isoharnstoffen oder Isothioharnstoffen der allgemeinen Formel III 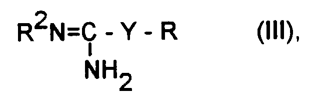 in der R, R² und Y die oben angegebene Bedeutung haben, in einem geeigneten inerten Lösungsmittel bei Raumtemperatur bis Rückflußtemperatur evtl. in Gegenwart einer Base, z. B. Triethylamin, umsetzt und gegebenenfalls anschließend Verbindungen der Formel I in andere Verbindungen der Formel I nachträglich umwandelt und anschließend chromatographisch bzw. durch Umkristallisation reinigt.

Die nachträgliche Umwandlung von Verbindungen der Formel I in andere Verbindungen der Formel I betrifft z. B. die Herstellung von Derivaten der allgemeinen Formel I, in denen X den Rest NR² darstellt. Diese Verbindungen werden hergestellt, indem man Verbindungen der allgemeinen Formel I, in denen X=S oder S Alkyl bedeutet, mit Aminen der Formel H₂NR² in einem polaren Lösungsmittel gegebenenfalls unter Druck oder in Gegenwart eines Carbodiimids bei Temperaturen zwischen 0°C und dem Siedepunkt des Lösungsmittels umsetzt.

Die Arzneimittel enthalten mindestens eine Verbindung der Formell zur Behandlung von viralen Infektionen und können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z. B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0.1-100 mg, vorzugsweise 0.2-80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0.5-500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2-1000 mg betragen. Der Wirkstoff kann auch durch Dauerinfusion gegeben werden, wobei die Mengen von 5-1000 mg pro Tag normalerweise ausreichen. Die Arzneimittel können auch mit anderen antiviral wirkenden Mitteln, wie z. B. AZT, kombiniert verabreicht werden.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage, die als racemische Gemische oder in optisch aktiver Form bzw. als reine R- und S-Enantiomere vorliegen können:
1. N-(Phenylaminothiocarbonyl)thiocarbamidinsäure-2-(4-methylpentyl)ester
2. N-(4-Chlorphenylaminothiocarbonyl)thiocarbamidinsäure-2-(4-methylpentyl)ester
3. N-(3-Fluorphenylaminothiocarbonyl)thiocarbamidinsäure-2-(4-methylpentyl)ester
4. N-(4-Dimethylaminophenylaminothiocarbonyl)thiocarbamidin-säure-2-(4-methylpentyl)ester
5. N-(4-Hydroxycarbonylphenylaminothiocarbonyl)thiocarbamidinsäure-2-(4-methylpentyl)ester
6. N-(4-Hydroxysulfonylphenylaminothiocarbonyl)thiocarbamidinsäure-2-(4-methylpentyl)ester
7. N-(4-Carboxyvinylcarbonylphenylaminothiocarbonyl)thio-carbamidinsäure-2-(4-methylpentyl)ester
8. N-(4-Methoxyphenylaminothiocarbonyl)thiocarbamidinsäure-2-(4-methylpentyl)ester
9. N-(3-Trifluormethylphenylaminothiocarbonyl)thiocarbamidin-säure-2-(4-methylpentyl)ester
10. N-(2-Bromphenylaminothiocarbonyl)thiocarbamidinsäure-2-(4-methylpentyl)ester
11. N-(4-Methylphenylaminothiocarbonyl)thiocarbamidinsäure-2-(4-methylpentyl)ester
12. N-(4-Nitrophenylaminothiocarbonyl)thiocarbamidinsäure-2-(4-methylpentyl)ester
13. N-(4-Isopropylphenylaminothiocarbonyl)thiocarbamidinsäure-2-(4-methylpentyl)ester
14. N-(3-Cyanophenylaminothiocarbonyl)thiocarbamidinsäure-2-(4-methylpentyl)ester
15. N-(4-Methylsulfonylphenylaminothiocarbonyl)thiocarbamidin-säure-2-(4-methylpentyl)ester
16. N-(2-Pyridylaminothiocarbonyl)thiocarbamidinsäure-2-(4-methylpentyl)ester
17. N-(3-Pyridylaminothiocarbonyl)thiocarbamidinsäure-2-(4-methylpentyl)ester
18. N-(4-Pyridylaminothiocarbonyl)thiocarbamidinsäure-2-(4-methylpentyl)ester
19. N-[(5-Brom-2-pyridyl)aminothiocarbonyl]-thiocarbamidinsäure-2-(4-methylpentyl) ester
20. N-(2-Thienylaminothiocarbonyl)-thiocarbamidinsäure-2-(4-methylpentyl)-ester
21. N-(4-Chlorphenylaminothiocarbonyl)thiocarbamidinsäure-methylester
22. N-(4-Chlorphenylaminothiocarbonyl)thiocarbamidinsäure-ethylester
23. N-(4-Chlorphenylaminothiocarbonyl)thiocarbamidinsäure-benzylester
24. N-(4-Chlorphenylaminothiocarbonyl)thiocarbamidinsäure-(2-chlorbenzyl)ester
25. N-(4-Chlorphenylaminothiocarbonyl)thiocarbamidinsäure-cyclohexylester
26. N-(4-Chlorphenylaminothiocarbonyl)thiocarbamidinsäure-(4(5)-imidazolylmethyl)ester
27. N-(4-Chlorphenylaminothiocarbonyl)thiocarbamidinsäure-hexylester
28. N-(4-Chlorphenylaminothiocarbonyl)thiocarbamidinsäure-(2-pyridylethyl)ester
29. N-(4-Chlorphenylaminothiocarbonyl)thiocarbamidinsäure-(3-chlorbenzyl)ester
30. N-(4-Chlorphenylaminothiocarbonyl)thiocarbamidinsäure-(4-chlorbenzyl)ester
31. N-(4-Chlorphenylaminothiocarbonyl)thiocarbamidinsäure-(2-aminoethyl)ester
32. N-(4-Chlorphenylaminothiocarbonyl)thiocarbamidinsäure-(2,5-dihydroxyphenyl)-ester
33. N-(4-Chlorphenylaminothiocarbonyl)thiocarbamidinsäure-(2-carboxyethyl)ester
34. N-(4-Chlorphenylaminothiocarbonyl)thiocarbamidinsäure-isopropentylester
35. N-(4-Chlorphenylaminothiocarbonyl)thiocarbamidinsäure-n-butylester
36. N-(4-Chlorphenylaminothiocarbonyl)thiocarbamidinsäure-(2-dimethylaminoethyl)-ester
37. N-(4-Chlorphenylaminothiocarbonyl)thiocarbamidinsäure-(3-dimethylaminopropyl)ester
38. N-(4-Chlorphenylaminothiocarbonyl)thiocarbamidinsäure-(2-pyridyl)ester
39. N-(4-Chlorphenylaminothiocarbonyl)thiocarbamidinsäure-(3-methyl-2-pyridyl)ester
40. N-(Phenylaminocarbonyl)thiocarbamidinsäure-methylester
41. N-(Phenylaminocarbonyl)thiocarbamidinsäure-benzylester
42. N-(Phenylaminocarbonyl)thiocarbamidinsäure-phenylester
43. N-(Phenylaminocarbonyl)thiocarbamidinsäure-2-(4-methylpentyl)ester
44. N-(Phenylaminothiocarbonyl)carbamidinsäure-ethylester
45. N-(Phenylaminothiocarbonyl)carbamidinsäure-allylester
46. N-(Phenylaminothiocarbonyl)carbamidinsäure-(3-methylphenyl)ester
47. N-(Phenylaminothiocarbonyl)carbamidinsäure-2-(5-methylpyridyl)ester

### Beispiel 1

### N-(4-Chlorphenylaminothiocarbonyl)thiocarbamidinsäure 2-(4-methylpentyl)ester

a) 22.83 g (0.3mol) Thioharnstoff und 55 g (0.33mol) 2-Brom-4-methylpentan werden 24 Stunden in 40 ml Ethanol am Rückfluß erhitzt. Das Lösungsmittel wurde anschließend eingeengt, der Rückstand mit 250 ml Ether versetzt und abgesaugt. Nach Umkristallisation aus Aceton erhält man 52 g 2-(4-methylpentyl)isothiuroniumbromid vom Smp. 143°C.
b) 2.65 g (11mmol) 2-(4-methylpentyl)isothiuroniumbromid (Bsp.1a), 1.69 g (10 mmol) 4-Chlorphenylisothiocyanat und 1.12 g (11 mmol) Triethylamin wurden in 20 ml Methylenchlorid 5 Stunden bei 25°C gerührt. Anschließend wurde die organische Phase mit kalter 1N Salzsäure und Hydrogencarbonatlösung gewaschen und eingeengt. Der Rückstand wurde aus Isopropanol/Isohexan (1:3) umkristallisiert. Man erhält 2.1 g der Titelverbindung vom Smp. 117-119°C.

### Beispiel 2

Analog Beispiel 1 erhält man die folgenden Verbindungen:
a) N-(Phenylaminothiocarbonyl)thiocarbamidinsäure 2-(4-methylpentyl)ester vom Smp. 95-96°C.
b) N-(3-Fluorphenylaminothiocarbonyl)thiocarbamidinsäure 2-(4-methylpentyl)ester vom Smp. 80-85°C.
c) N-(4-Chlorphenylaminothiocarbonyl)thiocarbamidinsäure methylester vom Smp. 145-146°C.
d) N-(4-Chlorphenylaminothiocarbonyl)thiocarbamidinsäure-ethylester vom Smp. 125-126°C
e) N-(4-Chlorphenylaminothiocarbonyl)thiocarbamidinsäure-benzylester vom Smp. 133-135°C
f) N-(4-Chlorphenylaminothiocarbonyl)thiocarbamidinsäure-cyclohexylester vom Smp 144-146°C
g) N-(4-Chlorphenylaminothiocarbonyl)thiocarbamidinsäure-3-chlorbenzylester vom Smp. 123-124°C
h) N-(4-Chlorphenylaminothiocarbonyl)thiocarbamidinsäure-4-chlorbenzylester vom Smp. 147-149°C
i) N-(4-Chlorphenylaminothiocarbonyl)thiocarbamidinsäure-hexylester vom Smp. 106-107°C
j) N-(4-Chlorphenylaminothiocarbonyl)thiocarbamidinsäure-butylester vom Smp. 126-128°C
k) N-(4-Chlorphenylaminothiocarbonyl)thiocarbamidinsäure-2-methylallylester vom Smp. 139-141°C
l) N-(4-Chlorphenylaminothiocarbonyl)thiocarbamidinsäure-2-(2-pyridyl)ethylester vom Smp. 132-134°C
m) N-(4-Chlorphenylaminothiocarbonyl)thiocarbamidinsäure-2-(4-morpholino)ethylester vom Smp. 121-123°C
n) N-(4-Chlorphenylaminothiocarbonyl)thiocarbamidinsäure-phenylester vom Smp. 127-129°C
o) N-(4-Chlorphenylaminothiocarbonyl)thiocarbamidinsäure-(4-methylpyridin-2yl)-methylester vom Smp. 148-149°C
p) N-[(4-Chlorphenyl)methylaminothiocarbonyl]thiocarbamidinsäure-2-(4-methylpentyl)ester als Öl. R_{f}-Wert: 0.7 (Toluol:Dioxan:Wasser)
q) N-[2-(4-Chlorphenyl)ethylaminothiocarbonyl]thiocarbamidinsäure-2-(4-methylpentyl)ester als Öl. R_{f}-Wert: 0.25 (Toluol)
r) N-(4-Aminosulfonylphenylaminothiocarbonyl)thiocarbamidinsäure-2-(4-methylpentyl)ester vom Smp. 113-116°C
s) N-(4-Carboxyphenylaminothiocarbonyl)thiocarbamidinsäure-2-(4-methylpentyl)ester vom Smp. 187-191°C
t) N-(Phenylaminothiocarbonyl)thiocarbamidinsäure-1-(2-methyl)phenethylester vom Smp. 84-86°C

### Beispiel 3

### N-(Phenylaminocarbonyl)thiocarbamidinsäure-2-(4-methylpentyl)ester

Analog Beispiel 1b erhält man aus 2.65 g (11mmol) 2-(4-Methyl-pentyl)isothiuroniumbromid, 1.19 g (10 mmol) Phenylisocyanat und 1.12 g (11 mmol) Triethylamin in 20ml Methylenchlorid 2.73 g der Titelverbindung als farbloses Harz (DC, Laufmittel: Toluol:Dioxan:Wasser (90:20:0.2), R_{f} = 0.64.

### Beispiel 4

### Antivirale Wirksamkeit

Die antivirale Wirksamkeit einiger ausgewählter Substanzen wurde sowohl in vivo als auch in vitro (Hemmung der reversen Transkriptase RT) untersucht.
a) MT2/MTT-Testsystem (in vivo)
MT2-Zellen werden mit HIV-1 (Isolat HTLV III B) infiziert. Nach 7 Tagen Inkubation erfolgt der MTT-Test, der die Vitalität der Zellen bestimmt. Die Vitalität der Zellen korreliert mit dem Ausmaß der Virusproduktion. In der folgenden Tabelle werden IC₅₀-Werte angegeben, d. h. diejenige Konzentration der Substanzen, die eine 50%-ige Inhibierung bewirken.
b) Hemmung der RT (in vitro)
In einem nicht radioaktiven Mikrotiterplattentest wird das Ausmaß der Inhibierung der Aktivität der RT bestimmt. Die Menge an eingebauten Nukleotiden in die zu synthetisierende DNA wird photometrisch bestimmt.

| IC₅₀-Werte (µg/ml) | | |
|---|---|---|
| **Bsp. Nr.** | **MT2** | **RT** |
| 1 | 0.6 | 0.6 |
| 2b | 3.3 | 1.0 |
| 2e | < 3 | 2.45 |
| 2f | < 3 | 0.103 |
| 2 g | < 3 | 0.33 |
| 2j | < 3 | 0.76 |
| 2n | - | 0.9 |

## Patentansprüche

1. Arzneimittel enthaltend als Wirkstoff mindestens ein (Thio)carbamidinsäure-Derivat der Formel I 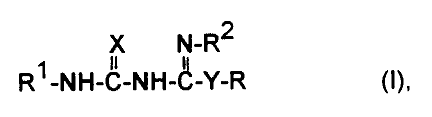 wobei
R einen geradkettigen oder verzweigtkettigen, gesättigten oder ungesättigten aliphatischen Rest mit 1-9 Kohlenstoffatomen, C₃-C₈-Cycloalkyl, C₁-C₆-Alkoxyalkyl, C₁-C₆- Alkylmercapto-C₁-C₆-alkyl, Amino-C₁-C₆-alkyl, C₁-C₆-alkylamino-C₁-C₆-alkyl, C₁-C₆-Dialkylamino-C₁-C₆-alkyl, Carboxy-C₁-C₆-alkyl, C₃-C₈-Cycloalkyl-C₁-C₆-alkyl; substituiertes Phenyl-C₁-C₆-alkyl; substituiertes Pyridyl-C₁-C₆-alkyl;
Phenyl, das gegebenenfalls ein- oder mehrfach durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁-C₆-Alkylcarbonylamino, C₁-C₆-Alkylaminocarbonyl, C₁-C₆-Alkoxycarbonyl, Hydroxy, Benzyloxy, Phenlymercapto, Phenyloxy, Nitro, Cyano, Halogen, Trifluormethyl, Azido, Phenlymercapto, Phenyloxy, Nitro, Cyano, Halogen, Trifluormethyl, Azido, Formylamino, Carboxy oder Phenyl substituiert sein kann;
einen carbocyclischen mono-, bi- oder tricyclischen Rest mit 7-15 C-Atomen oder ein heterocyclisches mono-, bi- oder tricyclisches Ringsystem darstellen kann, wobei die ungestättigten bzw. aromatischen Carbo- und Heterocyclen partiell oder vollständig hydriert sein können,
R¹ Phenyl, Phenyl-C₁-C₆-alkyl, Hetaryl oder Hetaryl-C₁-C₆-alkyl sein kann, wobei die Heteroatome gleich oder verschieden sein können und Sauerstoff, Schwefel oder Stickstoff bedeuten, die gegebenenfalls ein- oder mehrfach durch einen geradkettigen oder verzweigten, gesättigten oder ungesättigten aliphatischen C₁-C₆- bzw. C₂-C₆-Alkylrest, C₁-C₆-Alkoxy, C₁-C₆-Alkylmercapto, C₁-C₆-Alkylsulfinyl, C₁-C₆-Alkylsul- fonyl, C₁-C₆-Alkylamino, C₁-C₆- Dialkylamino, C₁-C₆-Alkoxycarbonyl, Carboxy, Carboxy-C₁-C₆-alkyl- carbonyl, Carboxy-C₂-C₆-alkenylcarbonyl, Amino, Halogen, Hydroxy, Nitro, Cyano, Azido, Phenyl oder Benzyloxy substituiert sein können,
R² Wasserstoff, C₁-C₆-Alkyl, C₁-C₆-Alkoxy, Cyano, Amino oder Hydroxy,
X Sauerstoff, Schwefel oder NR² und
Y Sauerstoff oder Schwefel bedeuten,
sowie deren Tautomere, Enantiomere, Diastereomere und physiologisch verträgliche Salze, mit der Maßgabe, daß R¹ nicht einen 2,6 - methylsubstituierten Phenylring darstellt, wenn X = O, Y = S, R = Methyl und R² = H ist.

2. Arzneimittel nach Anspruch 1, dadurch gekennzeichnet, daß R einen mono-, bi- oder tricyclischen carbocyclischen Ring mit 7-15 C-Atomen darstellt ausgewählt aus der Gruppe Naphthyl-, Anthracenyl-, Phenanthrenyl-, Flourenyl-, Indenyl-, Acenaphthylenyl-, Norbornyl-, Adamantyl-, C₃-C₇-Cyclo- alkyl- oder C₅-C₈-Cycloalkenylring, wobei der carbocyclische Ring unsubstituiert oder substituiert ist durch einen oder mehrere Substituenten aus der Gruppe C₁-C₆-Alkyl, C₁-C₆- Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₃-C₆-Alkenyloxy, C₁-C₆-Alkylamino, C₁-C₆-Dialkylamino-, C₁-C₆-Alkylcarbonylamino, C₁-C₆-Alkylaminocarbonyl, C₁-C₆-Alkoxycarbonyl-, Amino, Hydroxy, Nitro, Azido, Trifluormethyl, Cyano oder Halogen.

3. Arzneimittel nach Anspruch 1, dadurch gekennzeichnet, daß R einen heterocyclischen mono-, bi- oder tricyclischen Ring bedeutet ausgewählt aus der Gruppe Pyridin-, Pyrimidin-, Pyridazin-, Pyrazin-, Triazin-, Pyrrol-, Pyrazol-, Imidazol-, Triazol-, Thiazol-, Oxazol-, Isoxazol-, Oxadiazol-, Furazan-, Furan-, Thiophen-, Indol-, Chinolin-, Isochinolin-, Cumaron-, Thionephthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei diese partiell oder vollständig hydriert sein können, und diese Ringe unsubstituiert oder substituiert sind durch einen oder mehrere mehrere Substituenten aus der Gruppe C₁-C₆-Alkyl, C₁-C₆- Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₃-C₆-Alkenyloxy, C₁-C₆-Alkylamino, C₁-C₆-Dialkylamino-, C₁-C₆-Alkylcarbonylamino, C₁C₆-Alkylaminocarbonyl, C₁-C₆-Alkoxycarbonyl-, Amino, Hydroxy, Nitro, Azido, Trifluormethyl, Cyano oder Halogen.

4. Arzneimittel nach Anspruch 3, dadurch gekennzeichnet, daß R einen Pyrrol, Imidazol, Furan, Thiophen, Pyridin, Pyrimidin, Thiazol, Triazin, Indol, Chinolin, Isochinolin, Cumaron, Thionaphthen, Benzimidazol, Chinazolin, Methylendioxybenzol, Ethylendioxybenzol, Carbazol, Acridin oder Phenothiazinring bedeutet, der ein- oder zweifach substituiert sein kann durch C₁-C₃-Alkyl, C₁-C₃-Alkoxy, C₁-C₃-Alkylmercapto, C₁-C₃- Alkylsulfinyl, C₁-C₃-Alkylsulfonyl, C₂-C₄-Alkenyl, C₂-C₃-Alkinyl, C₃-C₄-Alkenyloxy, C₁-C₃-Alkylamino, C₁-C₃-Dialkylamino-, C₁-C₃-Alkylcarbonylamino, C₁-C₃-Alkylaminocarbonyl, C₁-C₃-Alkoxycarbonyl-, Amino, Hydroxy, Nitro, Azido, Trifluormethyl, Cyano oder Halogen.

5. Arzneimittel nach Anspruch 1, dadurch gekennzeichnet, daß R C₃-C₆-Alkyl, C₂-C₆-Alkenyl, C₂-C₄-Alkinyl, Benzyl, Phenethyl, Pyridylmethyl, Pyridylethyl, gegebenenfalls mono-oder disubstituiert durch C₁-C₃-Alkyl, C₁-C₃-Alkoxy oder Halogen; Phenyl, gegebenfalls substituiert durch C₁-C₃-Alkyl, C₁-C₃-Alkoxy, C₁-C₃-Alkylmercapto, Allyl, Allyloxy, C₁-C₃-Alkylamino, Di-C₁-C₃-alkylamino, Amino, Hydroxy, Azido, Trifluormethyl, Cyano oder Halogen bedeutet.

6. Arzneimittel nach den Ansprüchen 1-5, dadurch gekennzeichnet, daß R² Wasserstoff, Amino oder Hydroxy bedeutet.

7. Arzneimittel nach den Ansprüchen 1-6, dadurch gekennzeichnet, daß X Sauerstoff oder Schwefel bedeutet.

8. Arzneimittel nach den Ansprüchen 1-7, dadurch gekennzeichnet, daß Y Schwefel bedeutet.

9. Verbindungen der Formel I 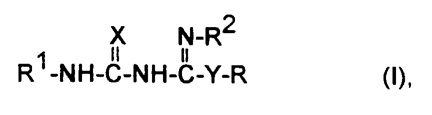 wobei
R geradkettiges oder verzweigtkettiges, gesättigtes oder ungesättigtes C₁-C₉- bzw. C₂-C₉-Alkyl, C₃-C₈-Cycloalkyl, C₁-C₆-Alkoxyalkyl, C₁-C₆-Alkylmercaptoalkyl, Aminoalkyl, C₁-C₆-alkylaminoalkyl, C₁-C₆-Dialkylaminoalkyl, Carboxyalkyl, C₃-C₈-Cycloalkylalkyl, substituiertes Phenyl- C₁-C₆-alkyl, substituiertes Pyridyl-C₁-C₆-alkyl, Phenyl, das gegebenenfalls ein- oder mehrfach durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁₋C₆-Alkylamino, Di-C₁-C₆-alkylamino, C₁-C₆-Alkylcarbonylamino, C₁-C₆-Alkylaminocarbonyl, C₁-C₆-Alkoxycarbonyl, Hydroxy, Benzyloxy, Phenlymercapto, Phenyloxy, Nitro, Cyano, Halogen, Trifluormethyl, Azido, Formylamino, Carboxy oder Phenyl substituiert sein kann, Naphthyl, Anthracenyl, Phenanthrenyl, Flourenyl, Indenyl, Acenaphthylenyl, Norbornyl, Adamantyl, C₃-C₇-Cycloalkyl, C₅-C₈-Cycloalkenyl oder ein heterocyclisches 1-, 2- oder 3-Ringsystem, wie das Pyridin-, Pyrimidin-, Pyridazin-, Pyrazin, Triazin-, Pyrrol-, Pyrazol-, Imidazol-,Triazol-, Thiazol-, Oxazol-, Isoxazol-, Oxadiazol-, Furazan-, Furan-, Thiphen-, Indol-, Chinolin-, Isochinolin-, Cumaron-, Thionaphthen-, Benzoxazol-, Benzthiazol-, Indazol, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin, Phenzin- oder Purinsystem, darstellen kann, wobei die ungesättigten bzw. aromatischen Carbo- und Heterocyclen partiell oder vollständig hydriert sein können,
R¹ Phenyl, Phenyl-C₁-C₆-alkyl, Hetaryl oder Hetaryl-C₁-C₆-alkyl sein kann, wobei die Heteroatome gleich oder verschie- den sein können und Sauerstoff, Schwefel oder Stickstoff bedeuten, die gegebenenfalls ein- oder mehrfach durch einen geradkettigen oder ver- zweigten, gesättigten oder ungesättigten C₁-C₆- bzw. C₂-C₆-Alkylrest, C₁-C₆-Alkoxy, C₁-C₆-Alkylmercapto, C₁-C₆-Alkylsulfinyl, C₁-C₆-Alkyl- sulfonyl, C₁-C₆-Alkylamino, C₁-C₆-Dialkylamino, C₁-C₆-Alkoxycarbonyl, Carboxy, Carboxyalkylcarbonyl, Carboxyalkenylcarbonyl, Amino, Halogen, Hydroxy, Nitro, Cyano, Azido, Phenyl oder Benzyloxy substituiert sein können,
R² Wasserstoff, C₁-C₆-Alkyl, C₁-C₆-Alkoxy, Cyano, Amino oder Hydroxy,
X Sauerstoff, Schwefel oder NR² und
Y Sauerstoff oder Schwefel bedeuten
sowie deren Tautomere, Enantiomere, Diastereomere und physiologisch verträgliche Salze,
mit der Maßgabe, daß R nicht C₁-C₄-Alkyl, Allyl, Benzyl, 4-Bromophenylmethyl oder Phenyl bedeutet, wenn R¹ einen unsubstituierten oder substituierten Phenylring darstellt.

10. Verfahren zur Herstellung von Verbindungen nach Anspruch 9, dadurch gekennzeichnet, daß man Isocyanate oder Thioisocyanate der allgemeinen Formel II
R¹ - N = C = X (II)
in der R¹ die oben angegebene Bedeutung hat und X Sauerstoff oder Schwefel ist, mit Isoharnstoffen oder Isothiohamstoffen der allgemeinen Formel III 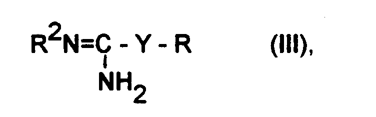 in der R, R² und Y die oben angegebene Bedeutung haben, in einem geeigneten inerten Lösungsmittel bei Raumtemperatur bis Rückflußtemperatur umsetzt und isoliert und gegebenenfalls anschließend Verbindungen der Formel I in andere Verbindungen der Formel I nachträglich umwandelt.

11. Verwendung von Verbindungen der Formel I nach Anspruch 1 oder 9 zur Herstellung von Arzneimitteln zur Behandlung von durch virale oder retrovirale Infektionen verursachten Erkrankungen.

## Claims

1. Pharmaceutical agent containing at least one (thio)carbimidinic acid derivative of formula I as the active substance 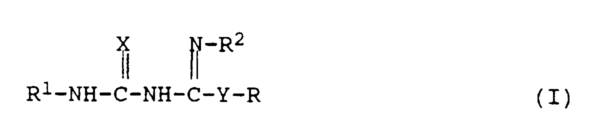 in which
R denotes a straight-chained or branched, saturated or unsaturated aliphatic residue with 1-9 carbon atoms, C₃-C₈ cycloalkyl, C₁-C₆ alkoxyalkyl, C₁-C₆ alkylmercapto-C₁-C₆-alkyl, amino-C₁-C₆-alkyl, C₁-C₆ alkylamino-C₁-C₆-alkyl, C₁-C₆ dialkylamino-C₁-C₆-alkyl, carboxy-C₁-C₆-alkyl, C₃-C₈ cycloalkyl-C₁-C₆-alkyl; substituted phenyl-C₁-C₆-alkyl; substituted pyridyl-C₁-C₆-alkyl;
phenyl which can be optionally substituted once or several times by C₁-C₆ alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₂-C₆ alkenyloxy, C₂-C₆ alkenylmercapto, C₂-C₆ alkinyloxy, C₂-C₆ alkinylmercapto, amino, C₁-C₆ alkylamino, di-C₁-C₆ alkylamino, C₁-C₆ alkylcarbonylamino, C₁-C₆ alkylaminocarbonyl, C₁-C₆ alkoxycarbonyl, hydroxy, benzyloxy, phenylmercapto, phenyloxy, nitro, cyano, halogen, trifluoromethyl, azido, formylamino, carboxy or phenyl;
can represent a carbocyclic monocyclic, bicyclic or tricyclic residue with 7-15 C atoms or a heterocyclic monocyclic, bicyclic or tricyclic ring system in which the unsaturated or aromatic carbocycles and heterocycles can be partially or completely hydrogenated,
R¹ can be phenyl, phenyl-C₁-C₆-alkyl, hetaryl or hetaryl-C₁-C₆-alkyl, wherein the heteroatoms can be the same or different and denote oxygen, sulphur or nitrogen which can be optionally substituted once or several times by a straight-chained or branched, saturated or unsaturated aliphatic C₁-C₆ or C₂-C₆ alkyl residue, C₁-C₆ alkoxy, C₁-C₆ alkylmercapto, C₁-C₆ alkyl-sulfinyl, C₁-C₆ alkylsulfonyl, C₁-C₆ alkyl-amino, C₁-C₆ dialkylamino, C₁-C₆ alkoxycarbonyl, carboxy, carboxy-C₁-C₆-alkylcarbonyl, carboxy-C₂-C₆-alkenylcarbonyl, amino, halogen, hydroxy, nitro, cyano, azido, phenyl or benzyloxy,
R² denotes hydrogen, C₁-C₆ alkyl, C₁-C₆ alkoxy, cyano, amino or hydroxy
X denotes oxygen, sulphur or NR² and
Y denotes oxygen or sulphur
as well as tautomers, enantiomers and diastereomers thereof and their physiologically tolerated salts, provided that R¹ does not represent a 2,6-methyl- substituted phenyl ring is X = O, Y = S, R = methyl and R² = H.

2. Pharmaceutical agent as claimed in claim 1, wherein R represents a monocyclic, bicyclic or tricyclic carbocyclic ring with 7-15 C atoms selected from the group comprising naphthyl, anthracenyl, phenanthrenyl, fluorenyl, indenyl, acenaphthylenyl, norbornyl, adamantyl, C₃-C₇ cycloalkyl or C₅-C₈ cycloalkenyl ring in which the carbocyclic ring can be unsubstituted or substituted by one or several substituents selected from the group comprising a C₁-C₆ alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₃-C₆ alkenyloxy, C₁-C₆ alkylamino, C₁-C₆ dialkyl-amino, C₁-C₆ alkylcarbonylamino, C₁-C₆ alkylamino-carbonyl, C₁-C₆ alkoxycarbonyl, amino, hydroxy, nitro, azido, trifluoromethyl, cyano or halogen.

3. Pharmaceutical agent as claimed in claim 1, wherein R denotes a heterocyclic monocyclic, bicyclic or tricyclic ring selected from the group comprising a pyridine, pyrimidine, pyridazine, pyrazine, triazine, pyrrole, pyrazole, imidazole, triazole, thiazole, oxazole, isoxazole, oxadiazole, furazan, furan, thiophene, indole, quinoline, isoquinoline, coumarone, thionaphthene, benzoxazole, benzthiazole, indazole, benzimidazole, benztriazole, chromene, phthalazine, quinazoline, quinoxaline, methylene-dioxybenzene, carbazole, acridine, phenoxazine, phenothiazine, phenazine or purine system in which these can be partially or completely hydrogenated and these rings are unsubstituted or substituted by one or several substituents selected from the group comprising C₁-C₆ alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₃-C₆ alkenyloxy, C₁-C₆ alkylamino, C₁-C₆ dialkylamino, C₁-C₆ alkylcarbonylamino, C₁-C₆ alkylaminocarbonyl, C₁-C₆ alkoxycarbonyl, amino, hydroxy, nitro, azido, trifluoromethyl, cyano or halogen.

4. Pharmaceutical agent as claimed in claim 3, wherein R denotes a pyrrole, imidazole, furan, thiophene, pyridine, pyrimidine, thiazole, triazine, indole, quinoline, isoquinoline, coumarone, thionaphthene, benzimidazole, quinazoline, methylenedioxybenzene, ethylenedioxybenzole, carbazole, acridine or phenothiazine ring which can be substituted once or twice by C₁-C₃ alkyl, C₁-C₃ alkoxy, C₁-C₃ alkylmercapto, C₁-C₃ alkylsulfinyl, C₁-C₃ alkylsulfonyl, C₂-C₄ alkenyl, C₂-C₃ alkinyl, C₃-C₄ alkenyloxy, C₁-C₃ alkylamino, C₁-C₃ dialkylamino, C₁-C₃ alkylcarbonylamino, C₁-C₃ alkylaminocarbonyl, C₁-C₃ alkoxycarbonyl, amino, hydroxy, nitro, azido, trifluoromethyl, cyano or halogen.

5. Pharmaceutical agent as claimed in claim 1, wherein R denotes C₃-C₆ alkyl, C₂-C₆ alkenyl, C₂-C₄ alkinyl, benzyl, phenethyl, pyridylmethyl, pyridylethyl which is optionally monosubstituted or disubstituted by C₁-C₃ alkyl, C₁-C₃ alkoxy or halogen; phenyl which is optionally substituted by C₁-C₃ alkyl, C₁-C₃ alkoxy, C₁-C₃ alkylmercapto, allyl, allyloxy, C₁-C₃ alkylamino, di-C₁-C₃ alkylamino, amino, hydroxy, azido, trifluoromethyl, cyano or halogen.

6. Pharmaceutical agent as claimed in claims 1-5, wherein R² denotes hydrogen, amino or hydroxy.

7. Pharmaceutical agent as claimed in claims 1-6, wherein X denotes oxygen or sulphur.

8. Pharmaceutical agent as claimed in claims 1-7, wherein Y denotes sulphur.

9. Compounds of formula I 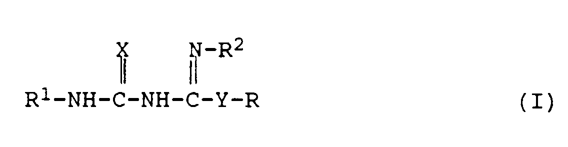 in which
R denotes a straight-chained or branched, saturated or unsaturated C₁-C₉ or C₂-C₉ alkyl, C₃-C₈ cycloalkyl, C₁-C₆ alkoxyalkyl, C₁-C₆ alkylmercaptoalkyl, aminoalkyl, C₁-C₆ alkylaminoalkyl, C₁-C₆ dialkylaminoalkyl, carboxyalkyl, C₃-C₈ cycloalkylalkyl; substituted phenyl-C₁-C₆-alkyl, substituted pyridyl-C₁-C₆-alkyl, phenyl which can be optionally substituted once or several times by C₁-C₆-alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₂-C₆ alkenyloxy, C₂-C₆ alkenylmercapto, C₂-C₆ alkinyloxy, C₂-C₆ alkinylmercapto, amino, C₁-C₆ alkylamino, di-C₁-C₆ alkylamino, C₁-C₆ alkylcarbonylamino, C₁-C₆ alkylaminocarbonyl, C₁-C₆ alkoxycarbonyl, hydroxy, benzyloxy, phenylmercapto, phenyloxy, nitro, cyano, halogen, trifluoromethyl, azido, formylamino, carboxy or phenyl; it can represent naphthyl, anthracenyl, phenanthrenyl, fluorenyl, indenyl, acenaphthylenyl, norbornyl, adamantyl, C₃-C₇ cycloalkyl, C₅-C₈ cycloalkenyl or a heterocyclic monocyclic, bicyclic or tricyclic system such as the pyridine, pyrimidine, pyridazine, pyrazine, triazine, pyrrole, pyrazole, imidazole, triazole, thiazole, oxazole, isoxazole, oxadiazole, furazan, furan, thiophene, indole, quinoline, isoquinoline, coumarone, thionaphthene, benzoxazole, benzthiazole, indazole, benzimidazole, benztriazole, chromene, phthalazine, quinazoline, quinoxaline, methylenedioxybenzene, carbazole, acridine, phenoxazine, phenothiazine, phenozine or purine system in which the unsaturated or aromatic carbocycles or heterocycles can be partially or completely hydrogenated.
R¹ can be phenyl, phenyl-C₁-C₆-alkyl, hetaryl or hetaryl-C₁-C₆ alkyl, wherein the heteroatoms can be the same or different and denote oxygen, sulphur or nitrogen, which can be optionally substituted once or several times by a straight-chained or branched, saturated or unsaturated C₁-C₆ or C₂-C₆ alkyl residue, C₁-C₆ alkoxy, C₁-C₆ alkylmercapto, C₁-C₆ alkylsulfinyl, C₁-C₆ alkylsulfonyl, C₁-C₆ alkylamino, C₁-C₆ dialkylamino, C₁-C₆ alkoxycarbonyl, carboxy, carboxyalkylcarbonyl, carboxyalkenylcarbonyl, amino, halogen, hydroxy, nitro, cyano, azido, phenyl or benzyloxy,
R² denotes hydrogen, C₁-C₆ alkyl, C₁-C₆ alkoxy, cyano, amino or hydroxy
X denotes oxygen, sulphur or NR² and
Y denotes oxygen or sulphur
as well as tautomers, enantiomers and diastereomers thereof and their physiologically tolerated salts,
provided that R does not denote C₁-C₄ alkyl, allyl, benzyl, 4-bromophenylmethyl or phenyl if R¹ represents an unsubstituted or substituted phenyl ring.

10. Process for the production of compounds as claimed in claim 9, wherein isocyanates or thioisocyanates of the general formula II
R¹ - N = C = X (II)
in which R¹ has the meaning stated above and X is oxygen or sulphur are reacted with isoureas or isothioureas of the general formula III 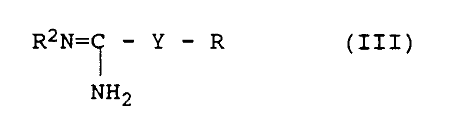 in which R, R² and Y have the meanings stated above, in a suitable inert solvent at room temperature to reflux temperature, they are isolated and subsequently compounds of formula I are optionally converted into other compounds of formula I.

11. Use of compounds of formula I as claimed in claim 1 or 9 for the production of pharmaceutical agents for the treatment of diseases caused by viral or retroviral infections.

## Revendications

1. Médicament contenant, en tant que principe actif, au moins un dérivé de l'acide (thio)carbamique, de formule I 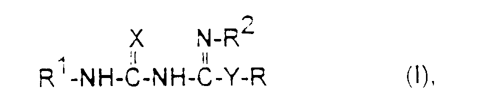 dans laquelle
R peut représenter un reste aliphatique saturé ou insaturé, à chaîne linéaire ou ramifié, comportant 1-9 atomes de carbone, un reste cycloalkyle en C₃-C₈, alcoxyalkyle en C₁-C₆, (alkyle en C₁-C₆)-mercapto(alkyle en C₁-C₆), aminoalkyle en C₁-C₆, (alkyle en C₁-C₆)-amino(alkyle en C₁-C₆), di(alkyle en C₁-C₆)-amino-(alkyle en C₁-C₆), carboxy(alkyle en C₁-C₆), cycloalkyle en C₃-C₈-(alkyle en C₁-C₆), phényl-(alkyle en C₁-C₆) substitué, pyridyl-(alkyle en C₁-C₆) substitué ;
un reste phényle pouvant être substitué une ou plusieurs fois par un groupe alkyle en C₁-C₆, alcoxy en C₁-C₆, alkylmercapto en C₁-C₆, alkylsulfinyle en C₁-C₆, alkylsulfonyle en C₁-C₆, alcényle en C₂-C₆, alcinyle en C₂-C₆, alcényloxy en C₂-C₆, alcénylmercapto en C₂-C₆, alcinyloxy en C₂-C₆, alcinylmercapto en C₂-C₆, amino, (alkyle en C₁-C₆)amino, di(alkyle en C₁-C₆)amino, (alkyle en C₁-C₆)carbonylamino, (alkyle en C₁-C₆)amino-carbonyle, (alcoxy en C₁-C₆)carbonyle, hydroxyle, benzyloxy, phénylmercapto, phényloxy, nitro, cyano, halogéno, trifluorométhyle, azido, formylamino, carboxy ou phényle ;
un reste carbocyclique mono-, bi- ou tricyclique ayant 7-15 atomes de C ou un système hétérocyclique mono-, bi- ou tricyclique, les composés carbocycliques et hétérocycliques insaturés ou aromatiques pouvant être partiellement ou totalement hydrogénés,
R¹ peut représenter un groupe phényle, phényle(alkyle en C₁-C₆), hétéroaryle ou hétéroaryle(alkyle en C₁-C₆), les hétéroatomes pouvant être identiques ou différents et pouvant signifier un atome d'oxygène, de soufre ou d'azote, qui peuvent être éventuellement une ou plusieurs fois substitués par un reste alkyle en C₁-C₆ ou C₂-C₆ aliphatique, saturé ou insaturé, à chaîne linéaire ou ramifiée, alcoxy en C₁-C₆, (alkyl en C₁-C₆)mercapto, alkylsulfinyle en C₁-C₆, alkylsulfonyle en C₁-C₆, alkylamino en C₁-C₆, di(alkyle en C₁-C₆)amino, (alcoxy en C₁-C₆)carbonyle, carboxy, carboxy(alkyle en C₁-C₆)carbonyle, carboxy(alcényle en C₂-C₆)carbonyle, amino, halogéno, hydroxyle, nitro, cyano, azido, phényle ou benzyloxy.
R² représente un atome d'hydrogène, un groupe alkyle en C₁-C₆, alcoxy en C₁-C₆, cyano, amino ou hydroxyle,
X représente un atome d'oxygène, de soufre ou NR², et
Y représente un atome d'oxygène ou de soufre,
ainsi que leurs tautomères, énantiomères, diastéréomères et leurs sels physiologiquement acceptables,
étant entendu que R¹ ne représente pas un noyau phényle substitué en positions 2,6 par un groupe méthyle, lorsque X=O, Y=S, R=méthyle et R²=H.

2. Médicament selon la revendication 1, caractérisé par le fait que R représente un noyau carbocyclique mono-, bi- ou tricyclique ayant 7-15 atomes de C, choisi dans l'ensemble englobant les groupes naphtyle, anthracényle, phénanthrényle, fluorényle, indényle, acénaphtényle, norbornyle, adamantyle, cycloalkyle en C₂-C₇ ou cycloalcényle en C₅-C₈, le noyau carbocyclique étant non substitué ou substitué par un ou plusieurs substituants choisis dans l'ensemble englobant les groupes alkyle en C₁-C₆, alcoxy en C₁-C₆, alkylmercapto en C₁-C₆, alkylsulfinyle en C₁-C₆, alkylsulfonyle en C₁-C₆, alcényle en C₂-C₆, alcinyle en C₂-C₆, alcényloxy en C₂-C₆, alkylamino en C₁-C₆, di(alkyle en C₁-C₆)amino, (alkyle en C₁-C₆)carbonylamino, (alkyle en C₁-C₆)aminocarbonyle, (alcoxy en C₁-C₆)carbonyle, amino, hydroxyle, nitro, azido, trifluorométhyle, cyano ou halogéno.

3. Médicament selon la revendication 1, caractérisé par le fait que R représente un noyau hétérocyclique mono-, bi- ou tricyclique, choisi dans l'ensemble englobant les groupes pyridine, pyrimidine, pyridazine, pyrazine, triazine, pyrrol, pyrazole, imidazole, triazole, thiazole, axazole, isoxazole, oxadiazole, furazane, furane, thiophène, indol, quinoléine, isoquinoléine, coumarone, thionaphtène, benzoxazole, benzothiazole, indazole, benzimidazole, benzotriazole, chromène, phtalazine, quinazoline, quinoxaline, méthylènedioxybenzène, carbazole, acridine, phénoxazine, phénothiazine, phénazine ou purine, ces groupes pouvant être partiellement ou totalement hydrogénés, et ces cycles étant substitués ou non substitués par un ou plusieurs substituants choisis dans l'ensemble englobant les groupes alkyle en C₁-C₆, alcoxy en C₁-C₆, alkylmercapto en C₁-C₆, alkylsulfinyle en C₁-C₆, alkylsulfonyle en C₁-C₆, alcényle en C₂-C₆, alcinyle en C₂-C₆, alcényloxy en C₃-C₆, alkylamino en C₁-C₆, di(alkyle en C₁-C₆)amino, (alkyle en C₁-C₆)carbonylamino, (alkyle en C₁-C₆)aminocarbonyle, (alcoxy en C₁-C₆)carbonyle, amine, hydroxyle, nitro, azido, tntluorométhyle, cyano ou halogéno.

4. Médicament selon la revendication 3, caractérisé par le fait que R représente un cycle pyrrole, imidazole, furane, thiophène, pyridine, pydimidine, thiazole, triazine, indol, chinoléine, isoquinoléine, coumarone, thionaphtène, benzimidazole, quinazoline, méthylènedioxybenzène, éthylènedioxybenzène, carbazole, acridine ou phénothiazine, qui peut être substitué une ou deux fois par un groupe alkyle en C₁-C₃, alcoxy en C₁-C₃, alkylmercapto en C₁-C₃, alkylsulfinyle en C₁-C₃, alkylsulfonyle en C₁-C₃, alcényle en C₂-C₄, alcinyle en C₂-C₃, alcényloxy en C₃-C₄, alkylamino en C₁-C₃, di(alkyle en C₁-C₃)amino, (alkyle en C₁-C₃)carbonylamino, (alkyle en C₁-C₃)aminocarbonyle, (alcoxy en C₁-C₃)carbonyle, amine, hydroxyle, nitro, azido, trilluoroinéthyle, cyano ou halogeno.

5. Médicament selon la revendication 1, caractérisé par le fait que R représente un groupe alkyle en C₃-C₆, alcényle en C₂-C₆, alcinyle en C₂-C₄, benzyle, phénéthyle, pyridylméthyle, pyridyléthyle, éventuellement mono- ou disubstitué par un groupe alkyle en C₁-C₃, alcoxy en C₁-C₃ ou halogéno ; phényle, éventuellement substitué par un groupe alkyle en C₁-C₃, alcoxy en C₁-C₃, (alkyle en C₁-C₃)mercapto, allyle, allyloxy, (alkyle en C₁-C₃)amino, di(alkyle en C₁-C₃)amino, amino, hydroxyle, azido, trifluorométhyle, cyano ou halogéno.

6. Médicament selon les revendications 1-5, caractérisé par le fait que R² représente un atome d'hydrogène, un groupe amino ou hydroxyle.

7. Médicament selon les revendications 1-6, caractérisé par le fait que X représente un atome d'oxygène ou de soufre.

8. Médicament selon les revendications 1-7, caractérisé par le fait que Y représente un atome de soufre.

9. Composés de formule générale I 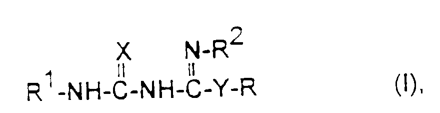 dans laquelle
R peut représenter un groupe alkyle en C₁-C₉ ou C₂-C₉ saturé ou insaturé, à chaîne linéaire ou ramifiée, cycloalkyle en C₃-C₈, alcoxyalkyle en C₁-C₆, (alkyle en C₁-C₆)mercaptoalkyle, aminoalkyle, (alkyle en C₁-C₆)aminoalkyle, di(alkyle en C₁-C₆)aminoalkyle, carboxyalkyle, (cycloalkyle en C₃-C₈)alkyle, phényl(alkyle en C₁-C₆) substitué, pyridyl(alkyle en C₁-C₆) substitué, un groupe phényle pouvant être éventuellement substitué une ou plusieurs fois par un groupe alkyle en C₁-C₆, alcoxy en C₁-C₆, (alkyle en C₁-C₆)mercapto, (alkyle en C₁-C₆)sulfinyle, (alkyle en C₁-C₆)-sulfonyle, alcényle en C₂-C₆, alcinyle en C₂-C₆, alcényloxy en C₂-C₆, (alcényle en C₂-C₆)mercapto, alcinyloxy en C₂-C₆, (alcinyle en C₂-C₆)mercapto, amino, (alkyle en C₁-C₆)-amino, di(alkyle en C₁-C₆)amino, (alkyle en C₁-C₆)carbonylamino, (alkyle en C₁-C₆)aminocarbonyle, (alcoxy en C₁-C₆)carbonyle, hydroxyle, benzyloxy, phénylmercapto, phényloxy, nitro, cyano, halogéno, trifluorométhyle, azido, formylamino, carboxyle ou phényle, un groupe naphtyle, anthracényle, phénantrényle, fluorényle, indényle, acénaphtylényle, norbornyle, adamantyle, cycloalkyle en C₂-C₇, cycloalcényle en C₅-C₈ ou un système hétérocyclique à 1, 2 ou 3 noyaux, tel qu'un système à base de pyridine, pyrimidine, pyridazine, pyrazine, triazine, pyrrol, pyrazole, imidazole, triazole, thiazole, oxazole, isoxazole, oxadiazole, furazane, furane, thiophène, indol, quinoléine, isoquinoléine, coumarone, thionaphtène, benzoxazole, benzothiazole, indazole, benzimidazole, benzotriazole, chromène, phtalazine, quinazoline, quinoxaline, méthylènedioxybenzène, carbazole, acridine, phénoxazine, phénothiazine, phénazine ou purine, les carbocycles ou hétérocycles insaturés ou aromatiques pouvant être partiellement ou totalement hydrogénés.
R¹ peut représenter un groupe phényle, phényl(alkyle en C₁-C₆), hétéroaryle ou hétéroaryle(alkyle en C₁-C₆), les hétéroatomes pouvant être identiques ou différents et représentant un atome d'oxygène, de soufre ou d'azote, qui peuvent être éventuellement substitués une ou plusieurs fois par un groupe alkyle en C₁-C₆ ou C₂-C₆ saturé ou insaturé, à chaîne linéaire ou ramifiée, alcoxy en C₁-C₆, (alkyle en C₁-C₆)mercapto, (alkyle en C₁-C₆)sulfinyle, (alkyle en C₁-C₆)-sulfonyle, (alkyle en C₁-C₆)-amino, di(alkyle en C₁-C₆)amino, (alcoxy en C₁-C₆)carbonyle, carboxyle, carboxyalkylcarbonyle, carboxyalcénylcarbonyle, amino, halogéno, hydroxyle, nitro, cyano, azido, phényle ou benzyloxy,
R² représente un atome dhydrogène, un groupe alkyle en C₁-C₆, alcoxy en C₁-C₆, cyano, amino ou hydroxyle,
X représente un atome d'oxygène, de soufre ou NR², et
Y représente un atome d'oxygène ou de soufre,
ainsi que leurs tautomères, énantiomères, diastéréomères et leurs sels physiologiquement acceptables,
étant entendu que R ne représente pas un groupe alkyle en C₁-C₄, allyle, benzyle, 4-bromophénylméthyle ou phényle lorsque R¹ représente un noyau phényle substitué ou non substitué.

10. Procédé de préparation des composés selon la revendication 9, caractérisé par le fait qu'un isocyanate ou thioisocyanate de formule générale II
R¹-N = C = X (II)
dans laquelle R¹ a les significations mentionnées et X représente un atome d'oxygène ou de soufre, est mis à réagir avec de l'isourée ou de l'isothiourée de formule générale III 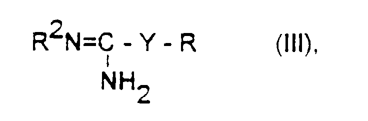 dans laquelle R, R² et Y ont les significations mentionnées,
dans un solvant inerte approprié, à une température comprise entre la température ambiante et la température du reflux, le produit est isolé et ensuite, les composés de formule I sont éventuellement transformés en d'autres composés de formule I.

11. Utilisation de composés de formule I selon la revendication 1 ou 9, pour la préparation de médicaments destinés au traitement de maladies provoquées par des infections virales ou rétrovirales.
